# EUROPEAN PATENT SPECIFICATION

(11) **EP 4 403 565 B1**
(45) Date of publication and mention of the grant of the patent: **29.04.2026**
(21) Application number: 22869220.8
(22) Date of filing: 13.09.2022
(51) Int. Cl.: C07J 41/00, A61P 25/00, A61P 25/08, A61P 25/20, A61P 25/24, A61P 25/28, A61K 31/57

(54) **WATER-SOLUBLE ALLOPREGNANOLONE DERIVATIVE, AND PREPARATION METHOD THEREFOR AND USE THEREOF**
WASSERLÖSLICHES ALLOPREGNANOLONDERIVAT UND HERSTELLUNGSVERFAHREN DAFÜR UND VERWENDUNG DAVON
DÉRIVÉ D'ALLOPRÉGNANOLONE HYDROSOLUBLE, PROCÉDÉ DE PRÉPARATION ASSOCIÉ ET UTILISATION CORRESPONDANTE

(30) Priority: 14.09.2021 CN 202111084187
(43) Date of publication of application: 24.07.2024
(73) Proprietor: Nanjing Minova Pharmaceutical Co., Ltd., Nanjing, Jiangsu 210046 (CN)
(72) Inventor: LIU, Fei, Nanjing, Jiangsu 210046 (CN); WU, Gang, Nanjing, Jiangsu 210046 (CN); WANG, Xiaobo, Nanjing, Jiangsu 210046 (CN); LIN, Chenggang, Nanjing, Jiangsu 210046 (CN); ZHAO, Xin, Nanjing, Jiangsu 210046 (CN); CHEN, Qing, Nanjing, Jiangsu 210046 (CN); LIU, Min, Nanjing, Jiangsu 210046 (CN)
(74) Representative: Gille Hrabal Partnerschaftsgesellschaft mbB Patentanwälte
(86) International application number: PCT/CN2022/118549
(87) International publication number: WO 2023/040851

(56) References cited:
- WO-A1-2011/120044
- WO-A2-2009/108804
- WO-A2-2010/088409
- CN-A- 108 148 106
- CN-A- 108 517 001
- FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1 January 1985 (1985-01-01), pages 1 - 40, XP009086953, ISSN: 0065-2490
- CHRISTOPHER J. MACNEVIN, FAHIM ATIF, IQBAL SAYEED, DONALD G. STEIN, DENNIS C. LIOTTA: "Development and Screening of Water-Soluble Analogues of Progesterone and Allopregnanolone in Models of Brain Injury", JOURNAL OF MEDICINAL CHEMISTRY, AMERICAN CHEMICAL SOCIETY, vol. 52, no. 19, 8 October 2009 (2009-10-08), pages 6012 - 6023, XP055046453, ISSN: 00222623, DOI: 10.1021/jm900712n

## Description

The present application claims priority to a prior application with the patent application No. 202111084187.4, entitled "WATER-SOLUBLE ALLOPREGNANOLONE DERIVATIVE, AND PREPARATION METHOD THEREFOR AND USE THEREOF" and filed with the China National Intellectual Property Administration on September 14, 2021.

### TECHNICAL FIELD

The present disclosure relates to the field of pharmaceuticals, and particularly relates to a water-soluble allopregnanolone derivative suitable for oral administration, a preparation method therefor and use thereof, and a pharmaceutical composition comprising same and use thereof in the prevention or treatment of central nervous system diseases, sedation and hypnosis, the treatment of Alzheimer's disease, the treatment of epilepsy or the treatment of depression, particularly postpartum depression.

### BACKGROUND

Neuroactive steroids are steroids active in nervous tissue and play an important regulatory role in humans. Neurosteroids mainly include progesterone, pregnenolone and the progesterone metabolite pregnanolone and the like. Neuroactive steroids play an important physiological role in humans, and impaired synthesis of these steroids in the body can lead to different neurological (CN 104736158 A) or psychiatric diseases (Expert Opin Ther Targets. 2014;18(6):679-90).

At present, pregnanolones are generally considered to include the following compounds: pregnanolone, allopregnanolone, epipregnanolone, and epiallopregnanolone (see Table 1).

**Table 1: The names of pregnanolones**

| Generic name | IUPAC name |
|---|---|
| Pregnanolone | 3α-OH-5β-pregnan-20-one |
| Allopregnanolone | 3α-OH-5α-pregnan-20-one |
| Epipregnanolone | 3β-OH-5β-pregnan-20-one |
| Epiallopregnanolone | 3β-OH-5α-pregnan-20-one |
| Tetrahydrodeoxycorticosterone | 3α-OH-5β-pregnan-21-ol-20-one |
| Allotetrahydrodeoxycorticosterone | 3α-OH-5α-pregnan-21-ol-20-one |

The patent document CN1300219A discloses that different pregnanolones have different mechanisms of action in the regulation of the central nervous system and have different physiological effects on the central nervous system. Allopregnanolone (3α-OH-5α-pregnan-20-one) is an important GABAA (γ-aminobutyric acid A) receptor and has the effect of combating epilepsy, helping with sleep, combating migraine and combating anxiety. Epiallopregnanolone (3β-OH-5α-pregnan-20-one) can block and antagonize the action of allopregnanolone, and properly control and terminate anesthesia caused by allopregnanolone and the like.

Allopregnanolone (3α-OH-5α-pregnan-20-one) has been a research hotspot in recent years. It was noted as early as 1986 that allopregnanolone is a positive modulator of the GABAA receptor. Allopregnanolone may bind primarily to α and β subunits of the GABAA receptor, increasing the opening frequency of the chloride ion channel on the receptor, decreasing nervous excitability, and thereby producing calming and anti-anxiety effects.

It has been reported that the levels of progesterone and its metabolites vary in different phases of the menstrual cycle. Before menstruation, the decreased levels of progesterone and its metabolites may cause premenstrual syndrome (PMS); that is, people may repeatedly experience some symptoms such as stress, anxiety and migraine before menstruation, and these symptoms will disappear after the menstrual cycle (Dalton, K., Premenstrual Syndrome and Progesterone Therapy, Second Edition, Chicago Yearbook, Chicago (1984)). Postpartum depression is also associated with abnormal levels of progesterone and its metabolites. The concentration of allopregnanolone in the plasma of healthy pregnant women increases as pregnancy progresses, and decreases dramatically after delivery. Studies have shown that the decrease in the allopregnanolone content is considered to be closely related to the development and progression of many mental disorders such as anxiety, depression, tremor, etc. Exogenous administration of allopregnanolone can significantly relieve the mental symptoms described above.

However, the existing allopregnanolone has low water solubility and poor oral bioavailability. It has a plasma half-life of about 45 minutes in the human body and can be rapidly metabolized, which is not conducive to making it into oral preparations.

The patent document CN104736158A discloses a method for formulating allopregnanolone with cyclodextrin to form a composition and administering it by intravenous infusion to treat epilepsy or persistent epilepsy. In the composition of allopregnanolone and cyclodextrin, the proportion of cyclodextrin is 1-30%, its plasma concentration is 50-2300 nM, and the treatment process is as long as 24 hours or more. However, cyclodextrin is a polymer compound and has some nephrotoxicity (Study on the Safety of the Medicinal Supramolecular Materials Cyclodextrin Derivatives, China Materials Science Technology and Equipment, 2009, Issue No. 5, pp. 1-3), which is a safety hazard. In 2019, an allopregnanolone injectable liquid developed by SAGE Therapeutics, Inc. was approved under the trade name ZULRESSO in the United States. The allopregnanolone injectable liquid is a sterile, transparent, colorless, preservative-free preparation for intravenous injection in which allopregnanolone and sulfobutylether-β-cyclodextrin sodium are made to form a clathrate to improve the solubility of allopregnanolone. The allopregnanolone injectable liquid is administered intravenously to produce stable, physiological concentrations of allopregnanolone for therapeutic effect. However, it requires a 60-hour complicated intravenous infusion, and during the infusion, continuous on-site monitoring by professional healthcare workers is required, along with necessary interventions. Consequently, patient compliance with the allopregnanolone injectable liquid is poor, and it is extremely inconvenient for healthcare workers to use. The method of infusing the allopregnanolone injectable liquid is as follows: 0-4 hours: infusion at a dose of 30 µg/kg/h; 4-24 hours: increasing the dose to 60 µg/kg/h; 24-52 hours: increasing the dose to 90 µg/kg/h (for patients unable to tolerate 90 µg/kg/h, a dose of 60 µg/kg/h could be considered); 52-56 hours: decreasing the dose to 60 µg/kg/h; 56-60 hours: decreasing the dose to 30 µg/kg/h.

WO2011120044A1 discloses modified neuroactive steroids which can be used to treat, prevent and/or ameliorating a phenotypic state of interest in a subject. CN108517001A and Christopher J. et al (Journal of Medicinal Chemistry, American Chemical Society, vol. 52, no. 19, 8, p 6012-6023) disclose water-soluble pregnenolone derivatives with improved solubility. WO2009108804A2 provides steroid analogues functionalized with polar substituents at the C3 and/or C20 positions of the steroid ring system that exhibit improved water solubility. WO2010088409A2 discloses a pharmaceutical composition comprising a neuroprotective steroid and vitamin D.

### SUMMARY

An object of the present disclosure is to provide an allopregnanolone derivative that is suitable for oral administration, has high bioavailability, acts fast, and can maintain a stable physiological concentration in vivo for a long time.

Another object of the present disclosure is to provide a composition comprising the allopregnanolone derivative and use of the allopregnanolone derivative described above for manufacturing a medicament for treating diseases caused by abnormality in the central nervous system.

The objects of the present disclosure are achieved by the following technical solutions.

In a first aspect, the present disclosure provides a compound of general formula I, a racemate thereof, a stereoisomer thereof, a tautomer thereof, a solvate thereof, a polymorph thereof or a pharmaceutically acceptable salt thereof: 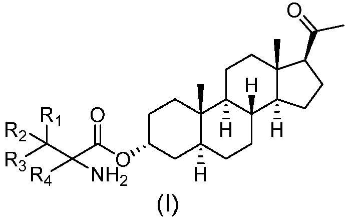 wherein:
the compound represented by formula I is the following structure:

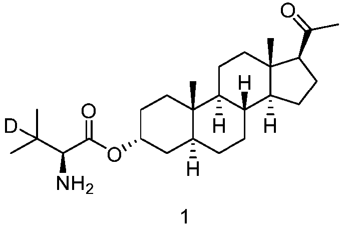

The present disclosure further provides a preparation method for the compound represented by formula I described above, comprising the following steps: 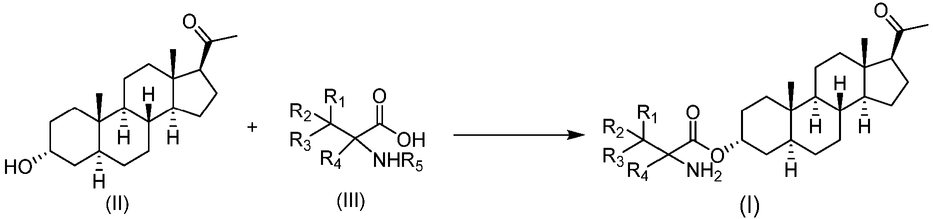
wherein R₁ and R₃ are CH₃, R₂ is D, R₄ is H, and R₅ is a protective group, , for example, an amino protecting group, such as tert-butyloxycarbonyl (Boc);
reacting the compound of formula II with a compound of formula III, and then removing a protective group to obtain the compound of formula I.

The present disclosure further provides a pharmaceutical composition, comprising the compound represented by formula I, the racemate thereof, the stereoisomer thereof, the tautomer thereof, the solvate thereof, the polymorph thereof or the pharmaceutically acceptable salt thereof.

According to the present disclosure, the pharmaceutical composition further comprises one or more pharmaceutically acceptable auxiliary materials.

According to the present disclosure, the pharmaceutical composition is for oral administration; the pharmaceutical composition may be a tablet, a pill, a lozenge, a dragee, a capsule, or the like.

The pharmaceutical composition of the present disclosure may be manufactured by methods well known in the art, such as conventional methods of mixing, granulation, manufacturing sugar-coated pills, and the like. For example, solid oral compositions may be prepared by a conventional method such as mixing, filling or tableting. For example, it may be obtained by the following method: mixing the active compound with a solid auxiliary material, optionally grinding the resulting mixture, adding other suitable auxiliary materials if desired, and processing the mixture into granules as the cores of tablets or dragees. Suitable auxiliary materials include, but are not limited to, adhesives, diluents, disintegrants, lubricants, glidants, sweeteners, flavoring agents, etc.

The present disclosure provides use of the compound of formula I, the racemate thereof, the stereoisomer thereof, the tautomer thereof, the solvate thereof, the polymorph thereof or the pharmaceutically acceptable salt thereof of the present disclosure, or the pharmaceutical composition of the present disclosure, for manufacturing a medicament for preventing or treating central nervous system diseases, for sedation and hypnosis, for treating Alzheimer's disease, for treating epilepsy, or for treating depression, particularly postpartum depression.

The present disclosure provides a method for preventing or treating central nervous system diseases, sedation and hypnosis, treating Alzheimer's disease, treating epilepsy or treating depression, particularly postpartum depression, comprising administering to an individual in need thereof a prophylactically or therapeutically effective amount of the compound of formula I, the racemate thereof, the stereoisomer thereof, the tautomer thereof, the solvate thereof, the polymorph thereof or the pharmaceutically acceptable salt thereof of the present disclosure, or the pharmaceutical composition of the present disclosure.

According to the present disclosure, the central nervous system diseases are, for example, traumatic brain injury, essential tremor, epilepsy (including refractory persistent epilepsy, rare genetic epilepsy (e.g., Dravet syndrome and Rett syndrome), depression (including postpartum depression), and Alzheimer's disease. The central nervous system diseases are selected from, for example, essential tremor, epilepsy, clinical depression, postnatal or postpartum depression, atypical depression, psychotic major depression, catatonic depression, seasonal affective disorder, dysthymia, double depression, depressive personality disorder, recurrent transient depression, minor depressive disorder, bipolar disorder or manic depressive disorder, post-traumatic stress disorder, depression caused by chronic medical conditions, treatment-resistant depression, refractory depression, suicidal tendency, suicidal ideations or suicidal behaviors.

In all methods of administering the compound of general formula I described herein, the daily dose is from 0.01 to 200 mg/kg body weight.

The dosing regimen may be adjusted to provide the optimum desired response. For example, a single oral dose may be administered, several separate doses may be administered over time, or the dose may be proportionally decreased or increased as indicated by the exigencies of the treatment situation. It is noted that dose values may vary with the type and severity of the condition to be alleviated, and may include single or multiple doses. It is further understood that for any particular individual, the specific dosage regimen will be adjusted over time according to the individual need and the professional judgment of the person administering the composition or supervising the administration of the composition.

### Definitions and Description

Unless otherwise stated, all technical and scientific terms used herein have the same meaning as commonly understood by those skilled in the art to which the present disclosure belongs..

Unless otherwise stated, the following definitions as used herein should be applied. When referring to a trade name, it is intended to refer to its corresponding commercial product or its active ingredient. As used herein, the terms "include", "comprise", "have", "contain", or "involve", and other variants thereof herein, are inclusive or open-ended and do not exclude other unrecited elements or method steps.

The term "preventing or treating" means administering a compound or preparation described herein to prevent, relieve or eliminate a disease or one or more symptoms associated with the disease, and includes:
(i) preventing the occurrence of a disease or a disease state in a mammal, particularly when the mammal is predisposed to the disease state but has not yet been diagnosed as having the disease state;
(ii) inhibiting the disease or the disease state, i.e., arresting its development; and
(iii) alleviating the disease or the disease state, i.e., causing regression of the disease or the disease state.

The term "deuteration ratio" refers to the ratio of the amount of isotope that labels synthesis to the amount of naturally occurring isotope. Unless otherwise stated, when a position in a structure is defined as H, i.e., hydrogen (H-1), that position contains only the naturally occurring amount of isotope. When a position in the structure is defined as D, i.e., deuterium (H-2), that position contains an amount of isotope that is at least 3340 times greater (i.e., at least 50.1% deuterium isotope) than the naturally occurring amount of isotope (0.015%).

The term "therapeutically effective amount" refers to an amount of the compound of the present disclosure that (i) treats or prevents a specified disease, condition, or disorder, (ii) reduces, relieves, or eliminates one or more symptoms of the specified disease, condition, or disorder, or (iii) prevents or delays the onset of one or more symptoms of the specified disease, condition, or disorder described herein. The amount of the compound of the present disclosure that constitutes a "therapeutically effective amount" varies depending on the compound, the disease state and its severity, the route of administration, and the age of the mammal to be treated, but may be routinely determined by those skilled in the art in view of their own knowledge and this disclosure.

The term "pharmaceutically acceptable" refers to those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt of the compound of the present disclosure includes salts thereof that are formed with pharmaceutically acceptable acids and salts thereof that are formed with pharmaceutically acceptable bases.

The term "pharmaceutically acceptable acids" as used herein refers to, for example, hydrochloric acid, hydrobromic acid, sulfuric acid, nitric acid, phosphoric acid, carbonic acid, formic acid, acetic acid, acetoacetic acid, trifluoroacetic acid, propionic acid, pyruvic acid, butyric acid, caproic acid, heptanoic acid, undecanoic acid, lauric acid, stearic acid, palmitic acid, oxalic acid, methanesulfonic acid, trifluoromethanesulfonic acid, ethanedisulfonic acid, isethionic acid, 1,5-naphthalenedisulfonic acid, 2-naphthalenesulfonic acid, camphorsulfonic acid, sulfamic acid, lactic acid, benzenesulfonic acid, p-toluenesulfonic acid, malonic acid, succinic acid, glutaric acid, adipic acid, maleic acid, fumaric acid, lactic acid, tartaric acid, citric acid, malic acid, benzoic acid, salicylic acid, cinnamic acid, naphthoic acid, pamoic acid, nicotinic acid, orotic acid, methylsulfuric acid, dodecylsulfuric acid, glutamic acid, aspartic acid, gluconic acid, glucuronic acid, or any combination thereof.

The term "pharmaceutically acceptable bases" as used herein refers to, for example, inorganic bases (alkali metal hydroxides or alkaline earth metal hydroxides, etc.) or organic bases (e.g., amines (primary, secondary or tertiary), etc.). Examples of suitable salts include, but are not limited to, organic salts obtained from amino acids, ammonia, primary amines, secondary amines, tertiary amines, and cyclic amines (e.g., diethylamine salts, piperidine salts, morpholine salts, piperazine salts, choline salts, meglumine salts, tromethamine salts, etc.), and inorganic salts obtained from sodium, calcium, potassium, magnesium, manganese, iron, copper, zinc, aluminum, and lithium.

The term "solvate" refers to a complex formed by coordination of the compound of the present disclosure in the solid or liquid state with solvent molecules. Hydrate is a particular form of solvate in which coordination occurs with water.

The term "pharmaceutical composition" refers to a mixture consisting of one or more of the compounds of the present disclosure or salts thereof and pharmaceutically acceptable auxiliary materials. The purpose of the pharmaceutical composition is to facilitate the administration of the compound of the present disclosure to an organism.

The term "pharmaceutically acceptable auxiliary materials" refers to those auxiliary materials which do not have a significant irritating effect on organisms and do not impair the bioactivity and performance of the active compound. Suitable auxiliary materials are well known to those skilled in the art, for example, carbohydrates, waxes, water-soluble and/or water-expandable polymers, hydrophilic or hydrophobic materials, gelatin, oils, solvents, water, and the like.

The pharmaceutical composition of the present disclosure may be prepared by combining the compound of the present disclosure with suitable pharmaceutically acceptable auxiliary materials. For oral administration, the pharmaceutical composition may be formulated by mixing the active compound with pharmaceutically acceptable auxiliary materials well known in the art. These auxiliary materials enable the compound of the present disclosure to be formulated as tablets, pills, lozenges, dragees, capsules and the like, for oral administration to patients.

As used herein, "individual" includes humans or non-human animals. Exemplary human individuals include human individuals with a disease (e.g., a disease described herein), which are referred to as patients, or normal individuals. In the present disclosure, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

### Beneficial Effects

The present disclosure provides an allopregnanolone derivative represented by formula I. By structurally modifying the hydroxyl group of allopregnanolone while preserving its pharmacological activity, an allopregnanolone derivative suitable for oral administration is obtained, and the derivative has good physical/chemical stability. It can release the active drug in vivo, thereby exerting pharmacological effects. The allopregnanolone derivative of the present disclosure can be stably metabolized and has good oral bioavailability and small toxic and side effects. It can quickly take effect after being orally administered and maintain a stable physiological concentration of allopregnanolone in vivo for a long time. It can be prepared into proper oral preparations, thereby improving the safety of medication and patient compliance and facilitating administration.

### BRIEF DESCRIPTION OF THE DRAWINGS

FIG. 1 shows the pharmacokinetic curves of allopregnanolone in the plasma of male rats after oral administration of the compounds of the present disclosure.
FIG. 2 shows the pharmacokinetic curves of allopregnanolone in the plasma of beagle dogs after oral administration of the compounds of the present disclosure.

### DETAILED DESCRIPTION

The general-formula compounds of the present disclosure, the preparation method therefor, and use thereof are described in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction scheme based on the existing embodiments.

The present disclosure is described in detail below through examples; however, these examples are not intended to limit the present disclosure in any way.

Experimental methods without specified conditions in the following examples are generally conducted under conventional conditions, or conditions recommended by the manufacturer. Unless otherwise indicated, the percentages and the number of parts are calculated by weight, and the starting materials and reagents used in the following examples are commercially available or may be manufactured by known methods.

The following abbreviations are used in the present disclosure: aq for aqueous solution; DMSO for dimethylsulfoxide; EtOAc for ethyl acetate; EtOH for ethanol; TFA for trifluoroacetic acid; i-PrOH for isopropanol; ECS for extracellular solution; ICS for intracellular solution; MI001 for allopregnanolone.

### Comparative Example 1: Preparation of Comparative Compound 1 (C1) Hydrochloride

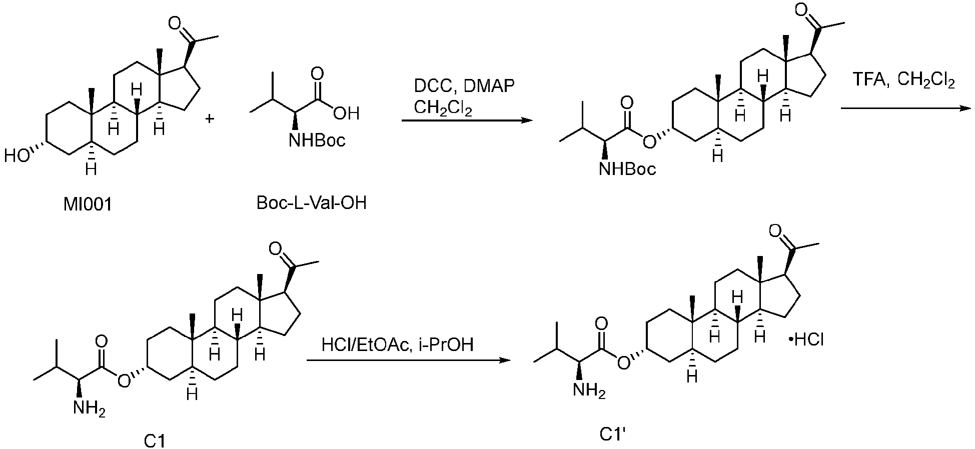

Step 1: MI001 (50.0 g, 157.0 mmol, 1.0 eq), Boc-L-Val-OH (tert-butoxycarbonyl L-valine) (40.9 g, 188.2 mmol), 4-dimethylaminopyridine (1.9 g, 15.5 mmol) and dichloromethane (500 mL) were added to a 1000 mL three-necked round-bottom reaction flask and stirred. The reaction system was cooled to -5 to 10 °C under nitrogen atmosphere. A solution of dicyclohexylcarbodiimide (38.9 g, 188.5 mmol) in dichloromethane (80 mL) was added dropwise, and the mixture was reacted at that temperature for 3 hours. After the reaction was complete as monitored by TLC (Thin Layer Chromatography), the reaction was stopped. The reaction liquid was filtered and the filter cake was washed with dichloromethane (100 mL). The filtrate was concentrated under reduced pressure, and the crude product was purified by column chromatography (petroleum ether (60-90)/ethyl acetate 20:1-10:1) on 100-200 mesh silica gel to give an off-white waxy solid (78.2 g, 96.2% yield).

Step 2: The product of step 1 (78 g, 150.6 mmol, 1.0 eq) and dichloromethane (320 mL) were added to a 1000 mL three-necked round-bottom reaction flask. Under nitrogen atmosphere, the system was magnetically stirred and cooled to 0-10 °C. Trifluoroacetic acid (171.8 g, 1506 mmol) was added dropwise and quickly. Then the mixture was reacted at 15-25 °C for 3 hours, and the reaction was stopped. The reaction liquid was poured into a solution of sodium bicarbonate (164.5 g, 1958 mmol) in water (780 mL) to quench the reaction. Dichloromethane (700 mL) was added, and the mixture was stirred and left to stand for liquid separation to give an organic phase. The organic phase was washed with 500 mL of purified water and dried over anhydrous sodium sulfate. After filtration and concentration, an off-white solid (59.5 g, 94.6% yield) was obtained.

Step 3: 0.5 g of the product described above was taken, and isopropanol (0.5 mL) and isopropyl acetate (7.5 mL) were added. After complete dissolution at room temperature, a hydrochloric acid-ethyl acetate solution (0.6 mL, 2.0 M hydrochloric acid-ethyl acetate) was added dropwise. The mixture was cooled to 5-10 °C, and a large amount of solid precipitated. The mixture was subjected to suction filtration, and the filter cake was dried to give a product (0.36 g, 66.5% yield, 99.90% HPLC purity).

¹H NMR (400 MHz, CDCl₃) *δ* 8.83 (brs, 3H), 5.23 - 5.14 (m, 1H), 4.00 - 3.88 (m, 1H), 2.52 (t, *J=* 8.7 Hz, 2H), 2.22 - 2.08 (m, 1H), 2.11 (s, 3H), 2.06 - 1.96 (m, 1H), 1.86 - 1.08 (m, 18H), 1.18 (m, 3H), 1.17 (m, 3H), 1.04 - 0.88 (m, 1H), 0.86 - 0.71 (m, 1H), 0.80 (s, 3H), 0.61 (s, 3H). MS m/z: 418.3 [M+H]⁺.

### Example 1: Synthesis of Compound 1 and Hydrochloride Thereof

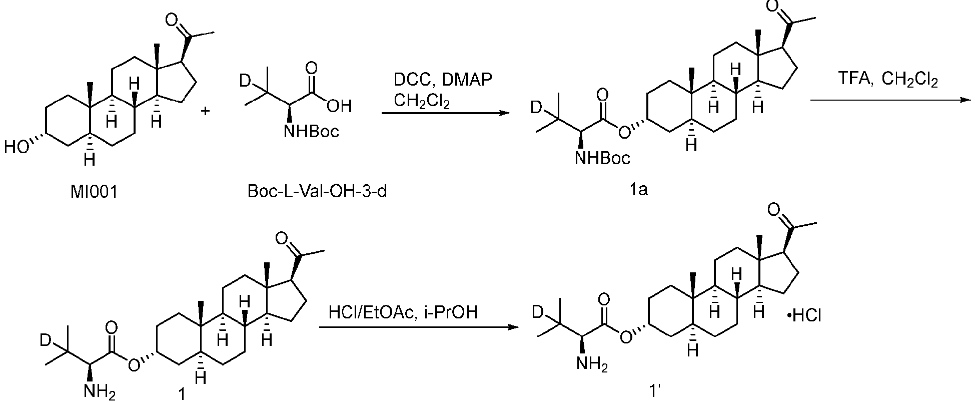

### Synthesis of intermediate 1a:

Boc-L-Val-OH-3-d (1.20 g, 5.5 mmol), MI001 (1.91 g, 6.0 mmol), 4-dimethylaminopyridine (0.07 g, 0.6 mmol), and dichloromethane (15 mL) were added to a 100 mL single-neck flask. A solution of dicyclohexylcarbodiimide (1.24 g, 6.0 mmol) in dichloromethane (5 mL) was added dropwise at 20 °C, and the mixture was stirred overnight. The dicyclohexylurea was removed by filtration. The filtrate was concentrated and purified by column chromatography (petroleum ether/ethyl acetate 20:1-7:1) to give intermediate 1a as a colorless oil (2.3 g, 80% yield).

¹H NMR (400 MHz, CDCl₃) *δ* 5.10 (m, 1H), 5.07 (d, *J =* 9.5 Hz, 1H), 4.23 (d, *J* = 9.1 Hz, 1H), 2.53 (t, *J =* 8.8 Hz, 1H), 2.26 - 2.08 (m, 1H), 2.11 (s, 3H), 2.06 - 1.95 (m, 1H), 1.85 - 0.71 (m, 20H), 1.46 (s, 9H), 0.98 (s, 3H), 0.90 (s, 3H), 0.80 (s, 3H), 0.61 (s, 3H).

### Synthesis of compound 1:

Intermediate 1a (2.3 g, 4.4 mmol) and dichloromethane (15 mL) were added to a 100 mL single-neck flask and dissolved with stirring at 20 °C. Trifluoroacetic acid (5.02 g, 44.0 mmol) was added dropwise. The mixture was stirred and reacted for 3-4 hours at a temperature of 15-25 °C and then dichloromethane (20 mL) was added. The reaction liquid was slowly poured into an aqueous sodium bicarbonate solution (15 g/50 mL) under stirring. After 5-15 minutes of stirring, the mixture was left to stand for liquid separation. The organic phase was washed with purified water (50 mL), separated, and dried over anhydrous sodium sulfate. After filtration and concentration, 1.73 g of compound 1 was obtained.

### Synthesis of compound 1 hydrochloride (1'):

The resulting compound 1 (1.59 g, 3.8 mmol) was dissolved in ethyl acetate (20 mL) and isopropanol (1.3 mL), and a HCl-ethyl acetate solution (2.4 M, 1.6 mL, 3.8 mmol) was added dropwise. The mixture was stirred at 20 °C for 1 hour and filtered. The filter cake was washed with ethyl acetate (20 mL). The mixture was dried at 40 °C under oil pump vacuum (P ≤ -0.09 MPa) for 4 hours to give a white solid (1.18 g, 68% yield).

¹H NMR (400 MHz, CDCl₃) *δ* 8.83 (brs, 3H), 5.23 - 5.14 (m, 1H), 4.00 - 3.88 (m, 1H), 2.52 (t, *J=* 8.7 Hz, 1H), 2.22 - 2.08 (m, 1H), 2.11 (s, 3H), 2.06 - 1.96 (m, 1H), 1.86 - 1.08 (m, 18H), 1.18 (s, 3H), 1.17 (s, 3H), 1.04 - 0.88 (m, 1H), 0.86 - 0.71 (m, 1H), 0.80 (s, 3H), 0.61 (s, 3H). MS m/z: 419.28 [M+H]⁺.

### Example 2: Synthesis of Compound 2 and Hydrochloride Thereof (not according to the present invention)

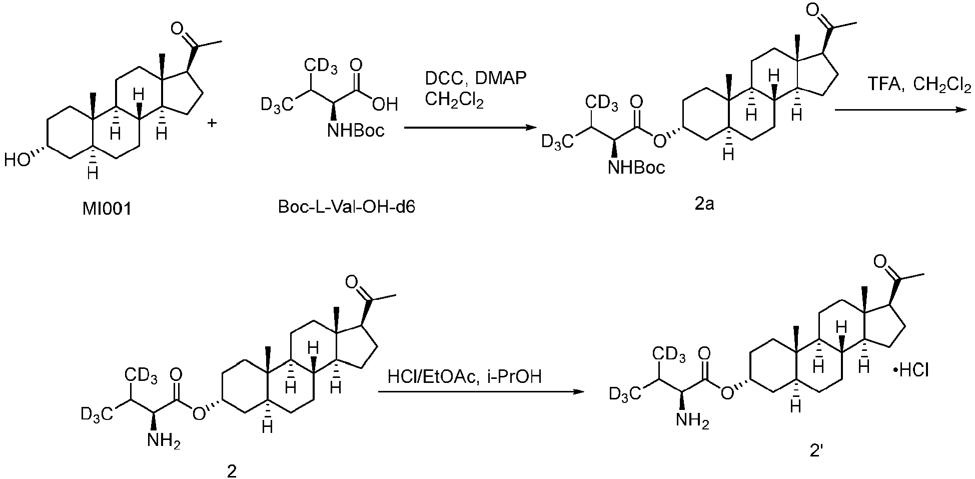

### Synthesis of intermediate 2a:

Under nitrogen atmosphere, Boc-L-Val-OH-d6 (2 g, 8.95 mmol), dichloromethane (30 g), MI001 (2.85 g, 8.95 mmol) and 4-dimethylaminopyridine (0.11 g, 0.90 mmol) were added to a 100 mL three-necked flask. The mixture was stirred and cooled to -5 °C to 5 °C. A solution of dicyclohexylcarbodiimide (2.1 g, 10 mmol) in dichloromethane (7.5 g) was added dropwise. After 3 hours of reaction at 15-25 °C, TLC monitoring showed the reaction was complete. The reaction liquid was washed with water, stirred and separated. The resulting organic phase was dried over anhydrous sodium sulfate, concentrated, and then separated by column chromatography (petroleum ether/ethyl acetate = 20:1) to give intermediate 2a as a colorless oil (3.2 g, 68.5% yield).

### Synthesis of compound 2:

The product 2a obtained from the previous step was dissolved in dichloromethane (16 mL), and trifluoroacetic acid (10.7 g) was added. After 3-4 hours of stirring at 15-25 °C, TLC monitoring showed the starting material had been completely consumed. The reaction liquid was added to an aqueous sodium bicarbonate solution (40 mL), and the pH was adjusted to 7-8. Dichloromethane (10 mL) was added, and after liquid separation, the organic phase was collected. The aqueous phase was extracted with dichloromethane (20 mL) once. The organic phases were combined, washed with water (5 mL × 3), dried over anhydrous sodium sulfate, and then concentrated to give a solid. The solid was triturated with acetonitrile (8 mL), and the triturate was filtered to give 1.2 g of compound 2.

### Synthesis of compound 2 hydrochloride (2'):

Compound 2 (0.6 g) was taken, and isopropanol (0.6 mL) and isopropyl acetate (9 mL) were added. After the compound was dissolved at room temperature, a hydrochloric acid-ethyl acetate solution (0.7 mL, a 2.0 M hydrochloric acid-ethyl acetate solution) was added dropwise. The mixture was cooled to 5-10 °C, and a large amount of solid precipitated. The mixture was subjected to suction filtration, and the filter cake was dried to give a product (0.3 g, 46.2% yield, 99.7% HPLC purity).

¹H NMR (400 MHz, CDCl₃) *δ* 8.85 (brs, 3H), 5.22 (s, 1H), 3.95 (brs, 1H), 2.54 (t, *J =* 8.7 Hz, 1H), 2.49 (s, 1H), 2.19 - 2.14 (m, 1H), 2.14 (s, 3H), 2.05 - 2.02 (m, 1H), 1.84 - 1.72(m, 5H), 1.61-1.41 (m, 7H), 1.32-1.17 (m, 6H), 1.04 - 0.88 (m, 1H), 0.85 (m, 1H), 0.82 (s, 3H), 0.63 (s, 3H). MS m/z: 424.39 [M+H]⁺.

### Example 3: Synthesis of Compound 3 and Hydrochloride Thereof (not according to the present invention)

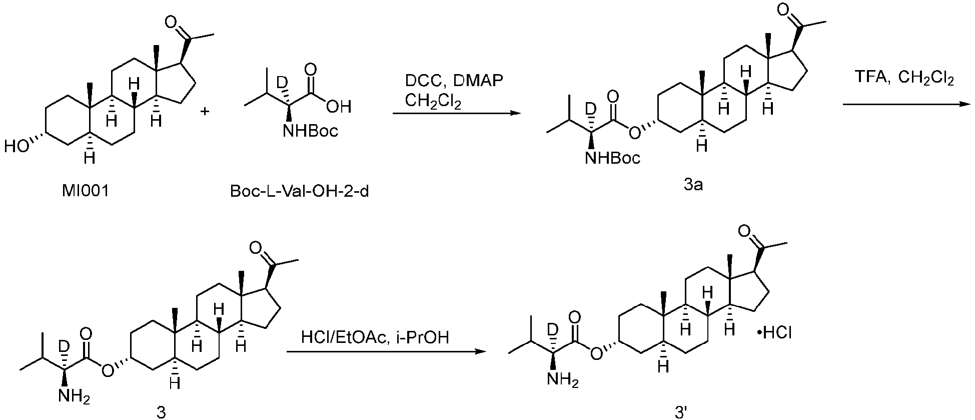

### Synthesis of intermediate 3a:

Boc-Val-OH-2-d (1.0 g, 4.6 mmol), MI001 (1.46 g, 4.6 mmol), 4-dimethylaminopyridine (0.06 g, 0.5 mmol), and dichloromethane (15 mL) were added to a 100 mL single-neck flask. A solution of dicyclohexylcarbodiimide (0.95 g, 4.6 mmol) in dichloromethane (5 mL) was added dropwise at 20 °C, and the mixture was stirred overnight. The mixture was filtered, and the filtrate was concentrated. The residue was purified by column chromatography (petroleum ether/ethyl acetate = 20:1-7:1) to give intermediate 3a as a colorless oil (1.6 g, 56% yield).

### Synthesis of compound 3:

The product intermediate 3a obtained from the previous step was dissolved in dichloromethane (8 g), and trifluoroacetic acid (4.5 g) was added. After 3-4 hours of stirring at 15-25 °C, TLC monitoring showed the starting material had been completely consumed. The reaction liquid was added to an aqueous sodium bicarbonate solution (20 mL), and the pH was adjusted to 7-8. Dichloromethane (5 mL) was added, and after liquid separation, the organic phase was collected. The aqueous phase was extracted with dichloromethane (10 mL) once. The organic phases were combined, washed with water (5 mL × 3), dried over anhydrous sodium sulfate, and then concentrated to give a solid. The solid was triturated with acetonitrile (4 mL), and the triturate was filtered to give 0.6 g of free base.

### Synthesis of compound 3 hydrochloride (3'):

The product compound 3 as described above (0.5 g) was taken, and isopropanol (0.5 mL) and isopropyl acetate (7.5 mL) were added. After the compound was dissolved at room temperature, a HCl-ethyl acetate solution (0.6 mL, a 2.0 M HCl-ethyl acetate solution) was added dropwise. The mixture was cooled to 5-10 °C, and a large amount of solid precipitated. The mixture was subjected to suction filtration, and the filter cake was dried to give a product (0.36 g, 66.5% yield, 99.70% HPLC purity).

¹H NMR (400 MHz, CDCl₃) *δ* 8.84 (brs, 3H), 5.21 (s, 1H), 2.56-2.48 (m, 2H), 2.21 - 2.17 (m, 1H), 2.13 (s, 3H), 2.05 - 2.02 (m, 1H), 1.83 - 1.36(m, 12H), 1.32-1.19 (m, 12H), 1.04 - 0.95 (m, 1H), 0.90-0.84(m, 1H), 0.82 (s, 3H), 0.63 (s, 3H). MS m/z: 419.25 [M+H]⁺.

### Example 4: Synthesis of Compound 4 and Hydrochloride Thereof (not according to the present invention)

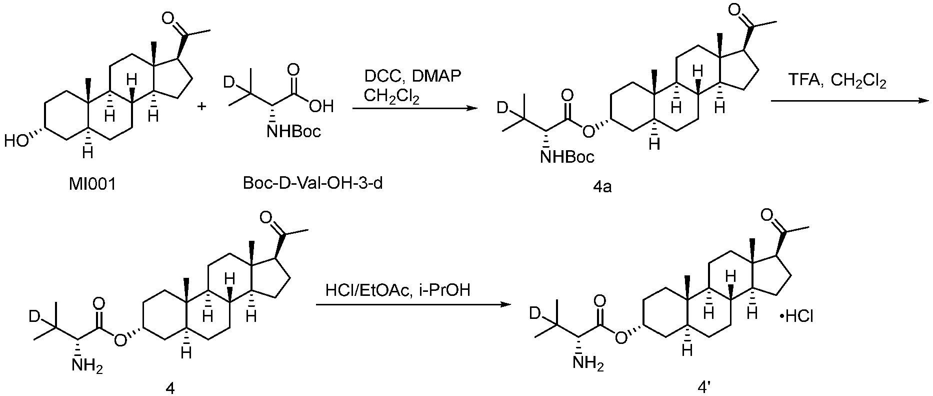

Compound 4 and compound 4 hydrochloride (4') were prepared with MI001 and Boc-D-Val-OH-3-d as the starting materials by the synthesis method of Example 1.

Compound 4 hydrochloride (4'), white solid, ¹H NMR (400 MHz, CDCl₃) δ 8.76 (brs, 3H), 5.16 (brs, 1H), 4.00 - 3.88 (m, 1H), 2.52 (t, J = 8.7 Hz, 1H), 2.22 - 2.08 (m, 1H), 2.08 (s, 3H), 2.06 - 1.96 (m, 1H), 1.86 - 1.08 (m, 18H), 1.18 (s, 3H), 1.17 (s, 3H), 1.04 - 0.88 (m, 1H), 0.86 - 0.71 (m, 1H), 0.79 (s, 3H), 0.61 (s, 3H). MS m/z: 419.35 [M+H]⁺.

### Example 5: Synthesis of Compound 5 and Hydrochloride Thereof (not according to the present invention)

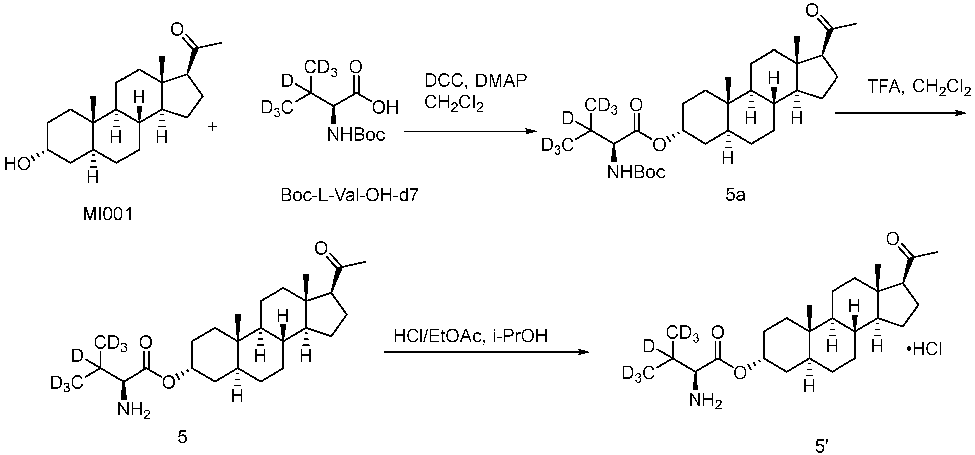

Compound 5 and compound 5 hydrochloride (5') were prepared with MI001 and Boc-D-Val-OH-d7 as the starting materials by the synthesis method of Example 2.

Compound 5 hydrochloride (5'), white solid, ¹H NMR (400 MHz, CDCl₃) *δ* 8.85 (brs, 3H), 5.22 (s, 1H), 3.95 (brs, 1H), 2.54 (t, *J =* 8.7 Hz, 1H), 2.19 - 2.14 (m, 1H), 2.14 (s, 3H), 2.05 - 2.02 (m, 1H), 1.84 - 1.72(m, 5H), 1.61-1.41 (m, 7H), 1.32-1.17 (m, 6H), 1.04 - 0.88 (m, 1H), 0.85 (m, 1H), 0.82 (s, 3H), 0.63 (s, 3H). MS m/z: 425.39 [M+H]⁺.

### Test Example 1: Solubility Experiment of Compounds

### 1. Sample preparation

Preparation of an external standard solution: 50 mg of a compound to be tested was precisely weighed out, added to a 10 mL volumetric flask, and dissolved in a proper amount of purified water by ultrasonication. The solution was diluted to volume and mixed well to give an external standard solution with a concentration of 5.0 mg/mL.

Preparation of a solution to be tested: 1.0 g of a compound to be tested was precisely weighed out and dissolved in 20 mL of purified water. The solution was stirred for dissolution for 24 h at 25 °C and centrifuged. The supernatant was taken and filtered through a 0.45 µm filter membrane, and the filtrate was collected. 1 mL of the filtrate described above was precisely measured out and added to a 5 mL measuring flask. Purified water was added to dilute the filtrate to volume, and the solution was mixed well to give a solution to be tested.

### 2. Determination of saturation solubility by external standard method

### Chromatographic conditions:

Chromatographic column: Waters XBridge C8 3.5 µm 4.6 × 100 mm NRT2019-21#, column temperature: 45 °C, detection wavelength: 205 nm;
Mobile phase A: a 10 mM/L (NH₄)₂HPO₄ solution, mobile phase B: acetonitrile, isocratic elution A:B = 40:60, flow rate: 1.0 mL/min;
Injection volume: 10 µL, run time: 10 min.

Purified water was used as a blank control solution, and the peak areas of the external standard solution and the standard solution were measured using a high performance liquid chromatograph with a UV detector and denoted as A_{external standard} and A_{to be tested}, respectively. The saturation solubility C of the compound to be tested was calculated by the following formula: C = A_{to be tested}/A_{external standard}*5 mg/mL*5, and the specific results are shown in Table 2 below.

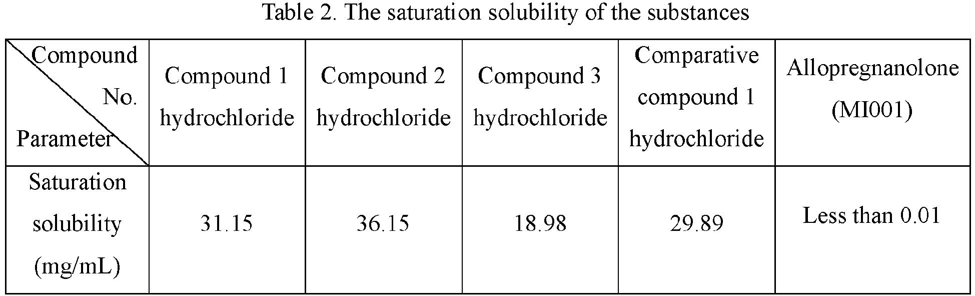

### Test Example 2: Results of Patch Clamp Experiment

The manual patch clamp method was used to measure the effect of the compounds on hERG potassium channel current stably expressed in Chinese hamster ovary cells. The inhibition of the drugs on the cardiac hERG potassium channel is the main cause of prolonged cardiac muscle repolarization. The larger the hERG IC₅₀ value, the lower the cardiotoxicity.

Experimental materials: experimental compounds (compound 1 prepared by the method of the present disclosure, and comparative compound 1), dimethylsulfoxide (Sigma-Aldrich (Shanghai) Trading Co., Ltd.), cisapride (positive control, commercially available), the Chinese hamster ovary (CHO) cell line, and CHO-hERG cells (Sophion Bioscience).

### Manual patch clamp assay method:

CHO-hERG cells in the exponential growth phase were collected and resuspended in ECS (extracellular solution) for later use. The cells were seeded in a cell recording chamber, and the chamber was placed on the stage of an inverted microscope. One of the cells in the tank was randomly selected for testing. The perfusion system was fixed onto the stage of the inverted microscope, and the cell was perfused continuously with ECS.

A recording microelectrode for the manual patch clamp assay was prepared using a capillary glass tube filled with intracellular solution. On the day of the patch clamp assay, an electrode was prepared using a borosilicate glass tube (GC150TF-10, Harvard Apparatus Co. UK). The resistance of the electrode, when filled with ICS, was between 2 and 5 MΩ.

The clamping voltage was -80 mV. First, the cell was depolarized to +60 mV, and the potential was maintained for 850 ms to open the hERG channel. The voltage was then set to -50 mV and maintained for 1275 ms, generating a bounce current, also known as a tail current. The peak value of the tail current was measured and used for analysis. Finally, the voltage was brought back to the clamping voltage (-80 mV). In the initial stage of recording the perfusion with the solvent control working solution, the peak value of the tail current was monitored until 3 or more scanning curves were stabilized, and then perfusion was performed with the test sample/positive control working solution to be tested until the inhibitory effect of the test sample/positive control working solution on the peak value of the hERG current reached a stable state.

The hERG current was recorded under the whole cell patch clamp technology at room temperature. The output signal of the patch clamp amplifier underwent a digital-to-analog conversion and 2.9 KHz low-pass filtering. Data were collected and recorded by Patchmaster Pro software and processed using Origin 8E software, and hERG IC₅₀ values were calculated. The experimental results are shown in Table 3 below:

**Table 3. The hERG activity of the compounds**

| Name of sample | hERG IC₅₀ value |
|---|---|
| Compound 1 | 1.93 µM |
| Comparative compound 1 | 0.77 µM |
| Cisapride | < 0.10 µM |

The inhibition of the cardiac hERG potassium channel by the drugs is the main cause of prolonged cardiac muscle repolarization. The half maximal inhibitory concentration (IC₅₀) value of compound 1 against hERG is 1.93 µM. Compound 1 of the present disclosure has lower inhibitory activity against hERG and smaller toxic and side effects on the heart than comparative compound 1.

### Test example 3: Metabolic Study

The metabolic stability of the example compound and the rate of production of the active substance allopregnanolone were judged in vitro (human liver microsome incubation system).

Experimental materials and reagents: human liver microsomes (Corning Inc., cat No. 452117); testosterone (Jiuding Chemicals Co., Ltd.); propafenone (Anpel Inc.); diclofenac, tolbutamide, acetonitrile, and DMSO from Sigma Co., Ltd.; NADPH (reduced coenzyme II) from Chem-Impex International Inc.; 0.1 M pH 7.4 PBS (phosphate-buffered saline, made in-house); other reagents were all analytically pure.

Instruments, conditions and parameters: liquid chromatography-mass spectrometry system (LC/MS/MS, Shimadzu LC 30-AD, MS API 4000); the chromatographic column was ACQUITY UPLC BEH C18 column (1.7 µm 2.1 × 50 mm Column, Part No. 186002350); the mobile phase was acetonitrile-water-formic acid (50:50:0.1); the flow rate was 0.7 mL/min; the injection volume was 5 µL; the column temperature was room temperature. An electrospray ionization (ESI) source was adopted with a spray voltage of 4.8 KV; the capillary temperature (TEM) was 300 °C; the sheath gas was N₂, and the flow rate was 10 psi; the auxiliary gas was N₂, and the flow rate was 1 psi; the collision-induced dissociation (CID) gas was Ar, and the pressure was 1.5 mTorr. The mass spectrometry scan mode was mass spectrometry multiple reaction monitoring (MRM), and the positive ion mode was used for detection. The internal standard was an acetonitrile solution containing 0.2 µg/mL tolbutamide; the lowest limit of quantification was 5 ng/mL, and the correlation coefficient was >0.99.

In vitro metabolic study method: The detection system was verified with testosterone, propafenone or diclofenac as a reference. Through an in vitro assay with the human liver microsome incubation system with allopregnanolone (compound MI001) and comparative compound 1 as references, the rate of reduction in the concentration of the example compound and the rate of MI001 generation were observed, and the in vitro metabolic stability of each example compound and the ability to maintain the MI001 concentration in liver microsomes were evaluated.

About 10 mg of each of the samples to be tested was precisely weighed out and dissolved in 0.1 mL of DMSO, and the solution was stepwise diluted with purified water to prepare 10 µM and 1 µM standard stock solutions. In an ice bath, the detection incubation system was prepared according to Table 3. NADPH was added to the incubation system (see Table 4 for its composition) to start a reaction, and 50 µL of the resulting mixture was taken immediately and added to 150 µL of acetonitrile as a time-zero sample and a 1 µM standard curve sample. Another 1 µM standard stock solution was added to the incubation system, and 50 µL of the resulting mixture was taken immediately and added to 150 µL of acetonitrile as a 0.1 µM standard curve sample. The remaining system was incubated in a water bath at 37 °C, and 50 µL of the remaining system was taken at 5 min, 15 min, 30 min, 1 h and 2 h and added to 150 µL of acetonitrile. Each sample was shaken and centrifuged at 18,000 g for 10 min, and a sample of the supernatant was taken and injected for LC/MS/MS analysis. The experimental results for some of the example compounds are shown in Table 5 below.

**Table 4. The composition of the incubation system**

| Component | Concentration |
|---|---|
| Liver microsome | 0.5 mg of protein/mL |
| Test Compound | 1 µM/10 µM |
| Control Compound | 1 µM |
| MgCl₂ | 1 mM |
| Acetonitrile | 0.99% |
| DMSO | 0.01% |
| MgCl₂ | 1 mM |

**Table 5. The concentrations (µM) of the compounds to be tested and MI001 measured at various time points**

| Compound No. | Detection indicator | Incubation time (min) | | | | | |
|---|---|---|---|---|---|---|---|
| | | 0 | 5 | 15 | 30 | 45 | 60 |
| Compound 1 | Compound 1 | 1.000 | 0.956 | 0.874 | 0.575 | 0.423 | 0.352 |
| | MI001 | NA | 0.034 | 0.060 | 0.111 | 0.113 | 0.125 |
| Comparative compound 1 | Comparative compound 1 | 1.000 | 0.801 | 0.6800 | 0.484 | 0.306 | 0.256 |
| | MI001 | NA | 0.042 | 0.091 | 0.132 | 0.196 | 0.214 |
| MI001 | MI001 | 1.000 | 0.626 | 0.414 | 0.259 | 0.150 | 0.119 |
| Testosterone | Testosterone | 1.000 | 0.677 | 0.379 | 0.155 | 0.063 | 0.044 |
| Propafenone | Propafenone | 1.000 | 0.851 | 0.597 | 0.232 | 0.025 | 0.004 |
| Diclofenac | Diclofenac | 1.000 | 0.374 | 0.077 | 0.008 | 0.001 | 0.001 |

The detection system was proved to be normal through the metabolism of testosterone, propafenone or diclofenac; the results for the comparative compound 1 and the example compound show that the compound of the present disclosure has good metabolic stability in human liver microsomes; the concentration of allopregnanolone resulting from the metabolism of the example compound in the liver microsome system rapidly reached a stable level, while comparative compound 1 had been unable to achieve a stable level.

### Test example 4: Pharmacokinetic Study

### 1. Pharmacokinetic study in SD rats

The purpose of this experiment was to study single oral administration of solutions of the compounds of the present disclosure and allopregnanolone solution to SD rats, to detect the active ingredient allopregnanolone in plasma, and to evaluate their pharmacokinetic (PK) profiles in SD rats.

Experimental materials: male SD rats (weighing 180-220 g, purchased from Beijing Vital River Laboratory Animal Technology Co., Ltd., production license No. SCXK(Beijing)2016-0006), experimental compounds (prepared according to the methods of the examples of the present disclosure), and purified water (made in-house).

Experimental method: Male SD rats were randomly grouped (3 rats per group), given ad libitum access to water during the assay, fasted for 12 or more hours before administration, and given food 4 hours after administration. These groups of SD rats were intragastrically administered, by mouth, solutions of the experimental compounds in 5% aqueous tween at a dose of 20 mg/kg (calculated based on allopregnanolone).

Blood samples were collected into K2EDTA anticoagulation tubes at 0 min before administration and at 5 min, 15 min, 30 min, 1 h, 2 h, 3 h, 4 h, 6 h, 8 h and 12 h after administration, and then were temporarily placed on ice before centrifugation.

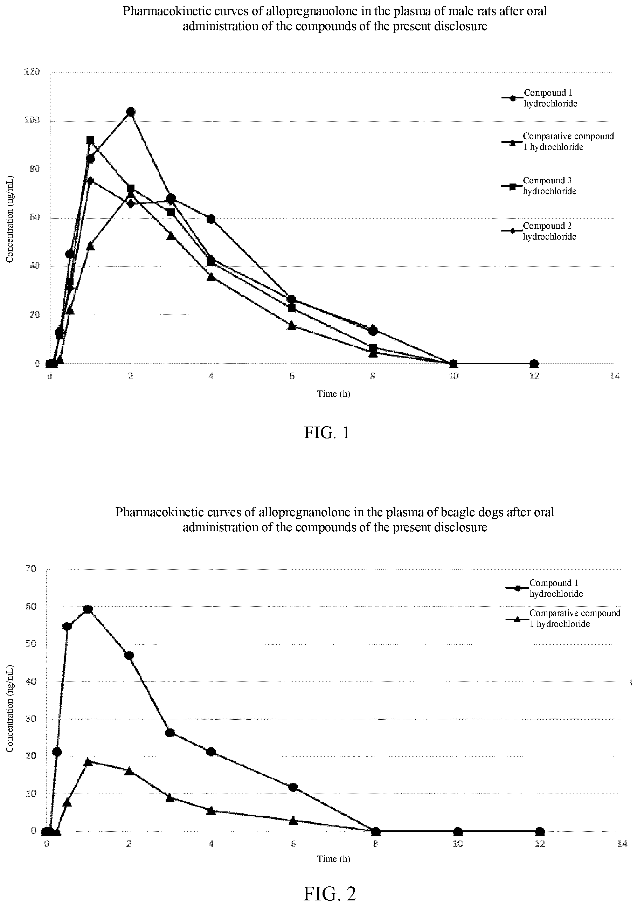

The blood was centrifuged within 60 min of being collected to isolate plasma (centrifuged at 8000 rpm at 2-8 °C for 5 min). After the centrifugation, the plasma was transferred to a 96-well plate or centrifuge tubes, transported in a box with ice, and stored at ≤-15 °C before LC-MS/MS analysis. The drug concentration in the plasma of SD rats was measured by LC-MS/MS bioanalysis, and the plasma concentration-time data were analyzed using WinNonlinTM (Version 8.3, Certara, USA) and a noncompartmental model to evaluate the pharmacokinetic (PK) profiles of the compounds in SD rats. The data are shown in Table 6, and the pharmacokinetic curves are shown in FIG. 1.

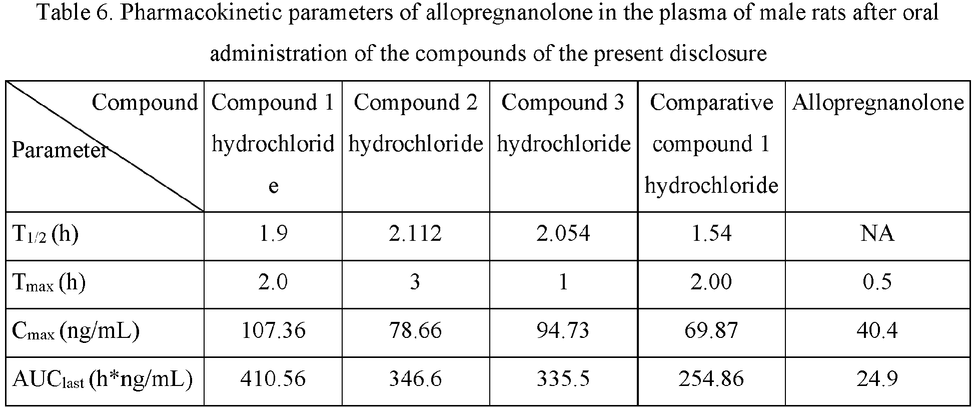

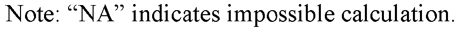

### 2. Pharmacokinetic study in beagle dogs

The inventors of the present disclosure have found through stability experiments with liver microsomes of different species that: the metabolism of the compounds of the present disclosure in the liver microsomes of different species is substantially similar, and the metabolic behavior in liver microsomes of beagle dogs is closest to that in liver microsomes of humans. The purpose of this experiment was to study single oral administration of solutions of the compounds of the present disclosure to beagle dogs, to detect the active ingredient allopregnanolone in plasma, and to evaluate their pharmacokinetic (PK) profiles in beagle dogs.

Experimental materials: male beagle dogs (weighing 6-15kg, purchased from Beijing Vital River Laboratory Animal Technology Co., Ltd.), experimental compounds (prepared according to the methods of the examples of the present disclosure), and purified water (made in-house).

Experimental method: Male beagle dogs were randomly grouped (3 rats per group), given ad libitum access to water during the assay, fasted for 12 or more hours before administration, and given food 4 hours after administration. These groups of beagle dogs were intragastrically administered, by mouth, solutions of the experimental compounds in 5% aqueous tween at a dose of 10 mg/kg (calculated based on allopregnanolone).

Blood samples were collected into K2EDTA anticoagulation tubes at 0 min before administration and at 5 min, 15 min, 30 min, 1 h, 2 h, 3 h, 4 h, 6 h, 8 h and 12 h after administration, and then were temporarily placed on ice before centrifugation.

The blood was centrifuged within 30 min of being collected to isolate plasma (centrifuged at 3200 rpm at 2-8 °C for 10 min). After the centrifugation, the plasma was transferred to a 96-well plate or centrifuge tubes, transported in a box with ice, and stored at ≤-60 °C before LC-MS/MS analysis. The drug concentration in the plasma of beagle dogs was measured by LC-MS/MS bioanalysis, and the plasma concentration-time data were analyzed using WinNonlin (Version 6.3 or later) and a noncompartmental model to evaluate the pharmacokinetic (PK) profiles of the compounds in beagle dogs. The data are shown in Table 7, and the pharmacokinetic curves are shown in FIG. 2.

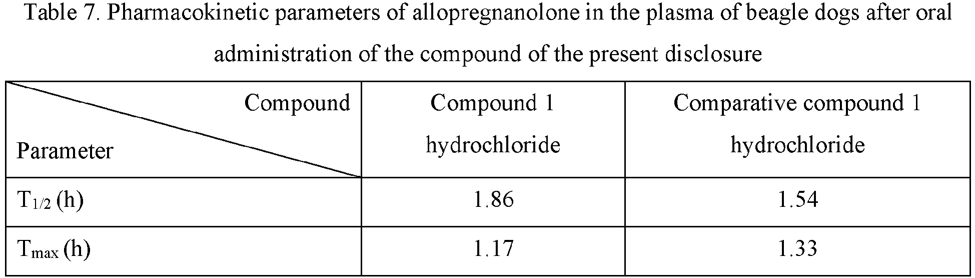

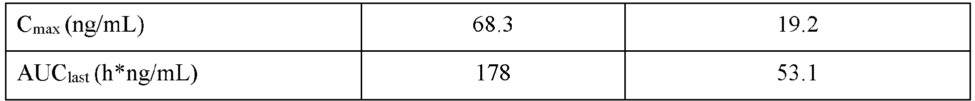

The above results show that the compound of the present disclosure has significantly improved pharmacokinetic properties; particularly, the AUC and Cmax were significantly improved after the compound of the present disclosure was administrated. The compound of the present disclosure is suitable for oral administration and can greatly overcome the defect that administration of allopregnanolone preparations for intravenous administration is time-consuming and needs to be continuously monitored by healthcare workers, thus greatly improving patient compliance and facilitating administration by healthcare workers.

The above examples illustrate the embodiments of the present disclosure. However, the present disclosure is not limited to the embodiments described above.

## Claims

1. A compound represented by formula I, a racemate thereof, a stereoisomer thereof, a tautomer thereof, a solvate thereof, a polymorph thereof or a pharmaceutically acceptable salt thereof: 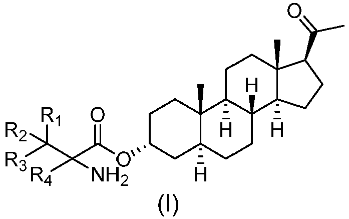 wherein the compound represented by formula I is the following structure:

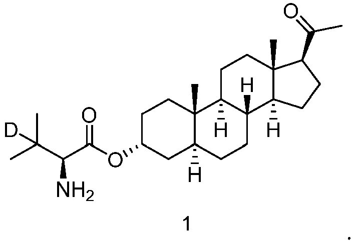

2. A preparation method for the compound according to claim 1, comprising the following steps: reacting the compound of formula II with a compound of formula III, and then removing a protective group to obtain the compound of formula I: 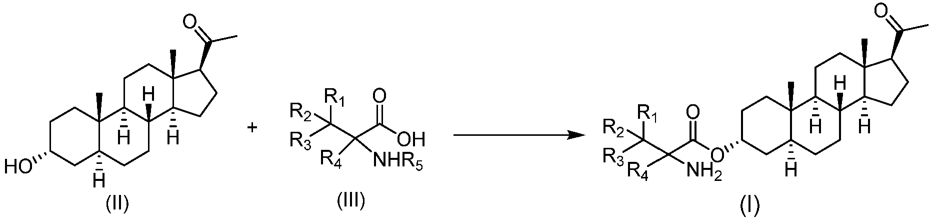 wherein R₁ and R₃ are CH₃, R₂ is D, R₄ is H, and R₅ is a protective group, such as Boc.

3. A pharmaceutical composition comprising the compound represented by formula I, the racemate thereof, the stereoisomer thereof, the tautomer thereof, the solvate thereof, the polymorph thereof or the pharmaceutically acceptable salt thereof according to claim 1.

4. The pharmaceutical composition according to claim 3, wherein the pharmaceutical composition further comprises one or more pharmaceutically acceptable auxiliary materials such as an adhesive, a diluent, a disintegrant, a lubricant, a glidant, a sweetener, a flavoring agent, etc.;

5. The pharmaceutical composition according to claim 3, wherein the pharmaceutical composition is for oral administration.

6. The pharmaceutical composition according to claim 3, wherein the pharmaceutical composition is a tablet, a pill, a lozenge, a dragee, a capsule, or the like.

7. Use of the compound of formula I or the racemate thereof, the stereoisomer thereof, the tautomer thereof, the solvate thereof, the polymorph thereof or the pharmaceutically acceptable salt thereof according to claim 1, or the pharmaceutical composition according to any one of claims 3-6 for manufacturing a medicament for preventing or treating central nervous system diseases, for sedation and hypnosis, for treating Alzheimer's disease, for treating epilepsy or for treating depression, particularly postpartum depression.

8. The use according to claim 7, wherein the central nervous system diseases are traumatic brain injury, essential tremor, epilepsy (including refractory persistent epilepsy, rare genetic epilepsy (e.g., Dravet syndrome and Rett syndrome)), depression (including postpartum depression), and Alzheimer's disease; the central nervous system diseases are selected from, for example, essential tremor, epilepsy, clinical depression, postnatal or postpartum depression, atypical depression, psychotic major depression, catatonic depression, seasonal affective disorder, dysthymia, double depression, depressive personality disorder, recurrent transient depression, minor depressive disorder, bipolar disorder or manic depressive disorder, post-traumatic stress disorder, depression caused by chronic medical conditions, treatment-resistant depression, refractory depression, suicidal tendency, suicidal ideations or suicidal behaviors.

## Patentansprüche

1. Verbindung, dargestellt durch Formel I, Racemat davon, Stereoisomer davon, Tautomer davon, Solvat davon, Polymorph davon oder pharmazeutisch verträgliches Salz davon: 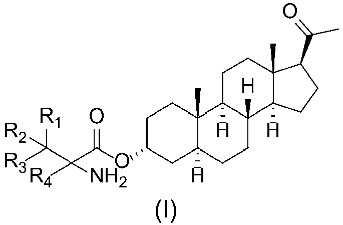 wobei die durch die Formel I dargestellte Verbindung die folgende Struktur ist:

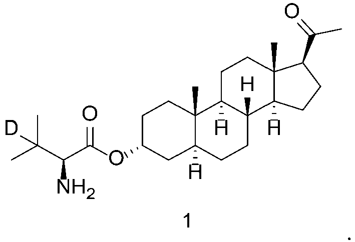

2. Zubereitungsverfahren für die Verbindung nach Anspruch 1, umfassend die folgenden Schritte: Umsetzen der Verbindung der Formel II mit einer Verbindung der Formel III und anschließendes Entfernen einer Schutzgruppe, um die Verbindung der Formel I zu erhalten: 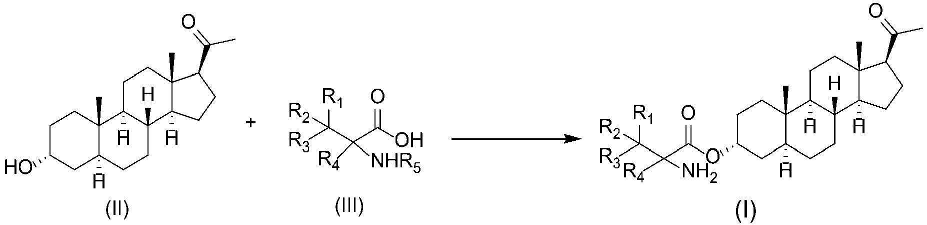 wobei R₁ und R₃ CH₃ sind, R₂ D ist, R₄ H ist und R₅ eine Schutzgruppe wie Boc ist.

3. Pharmazeutische Zusammensetzung, umfassend die Verbindung, dargestellt durch Formel I, das Racemat davon, das Stereoisomer davon, das Tautomer davon, das Solvat davon, das Polymorph davon oder das pharmazeutisch verträgliche Salz davon nach Anspruch 1.

4. Pharmazeutische Zusammensetzung nach Anspruch 3, wobei die pharmazeutische Zusammensetzung ferner ein oder mehrere pharmazeutisch verträgliche Hilfsmaterialien wie ein Haftmittel, ein Verdünnungsmittel, ein Sprengmittel, ein Schmiermittel, ein Gleitmittel, ein Süßungsmittel, einen Aromastoff usw. umfasst.

5. Pharmazeutische Zusammensetzung nach Anspruch 3, wobei die pharmazeutische Zusammensetzung zur oralen Verabreichung bestimmt ist.

6. Pharmazeutische Zusammensetzung nach Anspruch 3, wobei die pharmazeutische Zusammensetzung eine Tablette, eine Pille, eine Lutschtablette, eine Dragee, eine Kapsel oder dergleichen ist.

7. Verwendung der Verbindung der Formel I oder des Racemats davon, des Stereoisomers davon, des Tautomers davon, des Solvats davon, des Polymorphs davon oder des pharmazeutisch verträglichen Salzes davon nach Anspruch 1 oder der pharmazeutischen Zusammensetzung nach einem der Ansprüche 3-6 zum Herstellen eines Arzneimittels zum Vorbeugen oder Behandeln von Erkrankungen des zentralen Nervensystems, zur Sedierung und Hypnose, zum Behandeln der Alzheimer-Krankheit, zum Behandeln von Epilepsie oder zum Behandeln von Depression, insbesondere postpartaler Depression.

8. Verwendung nach Anspruch 7, wobei die Erkrankungen des zentralen Nervensystems traumatische Hirnverletzung, essentiellen Tremor, Epilepsie (einschließlich refraktärer persistierender Epilepsie, seltener genetischer Epilepsie (z. B. Dravet-Syndrom und Rett-Syndrom)), Depression (einschließlich postpartaler Depression) und Alzheimer-Krankheit sind; wobei die Erkrankungen des zentralen Nervensystems zum Beispiel ausgewählt werden aus essentiellem Tremor, Epilepsie, klinischer Depression, postnataler oder postpartaler Depression, atypischer Depression, psychotischer schwerer Depression, katatonischer Depression, saisonaler affektiver Störung, Dysthymie, doppelter Depression, depressiver Persönlichkeitsstörung, rezidivierender vorübergehender Depression, leichter depressiver Störung, bipolarer Störung oder manischdepressiver Störung, posttraumatischer Belastungsstörung, durch chronische medizinische Zustände verursachter Depression, behandlungsresistenter Depression, refraktärer Depression, suizidaler Neigung, suizidalen Gedanken oder suizidalen Verhaltensweisen.

## Revendications

1. Composé représenté par la formule I, un racémate de celui-ci, un stéréoisomère de celui-ci, un tautomère de celui-ci, un solvate de celui-ci, un polymorphe de celui-ci ou un sel pharmaceutiquement acceptable de celui-ci : 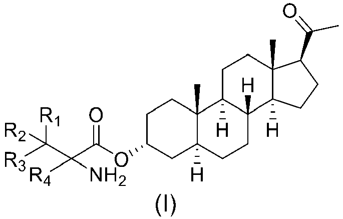 dans lequel le composé représenté par la formule I est la structure suivante :

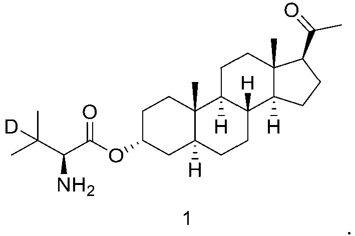

2. Procédé de préparation du composé selon la revendication 1, comprenant les étapes suivantes : la mise en réaction du composé de formule II avec un composé de formule III, puis l'élimination d'un groupe protecteur pour obtenir le composé de formule I : 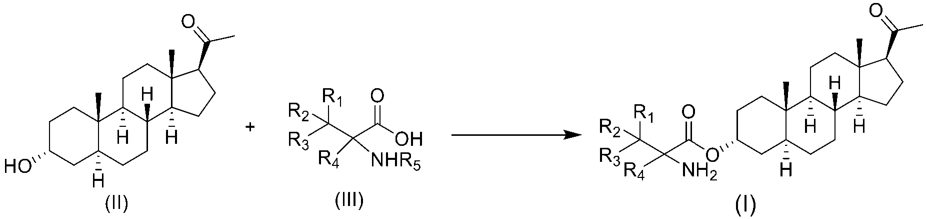 dans lequel R₁ et R₃ sont CH₃, R₂ est D, R₄ est H et R₅ est un groupe protecteur, tel que Boc.

3. Composition pharmaceutique comprenant le composé représenté par la formule I, le racémate de celui-ci, le stéréoisomère de celui-ci, le tautomère de celui-ci, le solvate de celui-ci, le polymorphe de celui-ci ou le sel pharmaceutiquement acceptable de celui-ci selon la revendication 1.

4. Composition pharmaceutique selon la revendication 3, dans laquelle la composition pharmaceutique comprend en outre un ou plusieurs matériaux auxiliaires pharmaceutiquement acceptables tels qu'un adhésif, un diluant, un délitant, un lubrifiant, un agent de glissement, un édulcorant, un agent aromatisant, etc.

5. Composition pharmaceutique selon la revendication 3, dans laquelle la composition pharmaceutique est destinée à l'administration orale.

6. Composition pharmaceutique selon la revendication 3, dans laquelle la composition pharmaceutique est un comprimé, une pilule, une pastille, une dragée, une capsule ou analogue.

7. Utilisation du composé de formule I ou du racémate de celui-ci, du stéréoisomère de celui-ci, du tautomère de celui-ci, du solvate de celui-ci, du polymorphe de celui-ci ou du sel pharmaceutiquement acceptable de celui-ci selon la revendication 1, ou de la composition pharmaceutique selon l'une quelconque des revendications 3 à 6 pour la fabrication d'un médicament destiné à prévenir ou à traiter des maladies du système nerveux central, à la sédation et à l'hypnose, à traiter la maladie d'Alzheimer, à traiter l'épilepsie ou à traiter la dépression, en particulier la dépression post-partum.

8. Utilisation selon la revendication 7, dans laquelle les maladies du système nerveux central sont les lésions cérébrales traumatiques, les tremblements essentiels, l'épilepsie (y compris l'épilepsie persistante réfractaire, les épilepsies génétiques rares (par exemple, le syndrome de Dravet et le syndrome de Rett)), la dépression (y compris la dépression post-partum) et la maladie d'Alzheimer ; les maladies du système nerveux central sont choisies parmi, par exemple, les tremblements essentiels, l'épilepsie, la dépression clinique, la dépression postnatale ou post-partum, la dépression atypique, la dépression majeure psychotique, la dépression catatonique, le trouble affectif saisonnier, la dysthymie, la dépression double, le trouble de la personnalité dépressive, la dépression transitoire récurrente, le trouble dépressif mineur, le trouble bipolaire ou le trouble maniaco-dépressif, le trouble de stress post-traumatique, la dépression provoquée par des problèmes de santé chroniques, la dépression résistante au traitement, la dépression réfractaire, la tendance suicidaire, les idées suicidaires ou les comportements suicidaires.
